# EUROPEAN PATENT SPECIFICATION

(11) **EP 0 337 502 B1**
(45) Date of publication and mention of the grant of the patent: **16.02.1994**
(21) Application number: 89109875.8
(22) Date of filing: 14.08.1984
(51) Int. Cl.: C12P 25/00

(54) **Process for the preparation of riboflavin**
Verfahren zur Herstellung von Riboflavin
Procédé de préparation de riboflavine

(30) Priority: 09.09.1983 JP 165245/83; 17.05.1984 JP 99096/84; 17.05.1984 JP 99097/84
(43) Date of publication of application: 18.10.1989
(62) Divisional of application: 84109683.7
(73) Proprietor: DAICEL CHEMICAL INDUSTRIES, LTD., Sakai-shi Osaka-fu 590 (JP)
(72) Inventor: Kawai, Kimitoshi, Himeji-shi Hyogo (JP); Matsuyama, Akinobu, Arai-shi, Niigata (JP); Takao, Shoichi, Sapporo-shi Hokkaido (JP)
(74) Representative: Grünecker, Kinkeldey, Stockmair & Schwanhäusser Anwaltssozietät

(56) References cited:
- GB-A- 553 465
- US-A- 2 449 142
- US-A- 2 493 274

## Description

### Field of the invention:

The present invention relates to a process for the preparation of riboflavin by fermentation.

Riboflavin can be prepared in a high yield by the fermentation method according to the present invention.
Riboflavin is of value for the applications as medical and feeding stuff additives or colorants for food.

### Description of the Prior Art:

Typical riboflavin-producing microorganisms known at present are Ascomycetes such as Ashbya gossypii or Eremothecium ashbyii, with which riboflavin for feeding stuff is industrially prepared from saccharides for the production of additives for feeding stuff.

It is known that riboflavin is produced not only by Ascomycetes but also by some bacteria belonging to the genus Clostridium or yeasts of the genus Candida, Saccharomyces, or Hansenula (see Progress in Industrial Micobiology, Vol. 1, p. 139 (1959)). However, the productivity of riboflavin is low when bacteria or yeasts are used and it is also noticed that the presence of iron ions in trace amounts greatly decreases the productivity. Because of these disadvantages, the production of riboflavin with bacteria or yeasts has not yet been put into industrial practice (see Annual Review of Microbiology, Vol. 26, p. 369 (1972)).

Some of the present inventors reported on a process for the preparation of riboflavin by fermentation using acetic acid as carbon source (see Takao, Agr. Biol. Chem, Vol. 28, pp. 559, 566 & 765 (1964)).

As to the recovery of riboflavin out of the culture, those for feeding stuff additives are obtained by drying the entire culture without isolation of riboflavin. In that case, a method of utilizing the difference in specific gravity is suggested in order to obtain as high riboflavin concentration as possible (see Japanese Patent Laid-Open No. 159800/1980).

Highly purified crystalline riboflavin, which can be used for medicaments and the like, has been obtained by heating the culture to solubilize riboflavin, separating unsoluble matters comprising microorganisms from the solution, and thereafter separating riboflavin from that solution by temporarily changing riboflavin into a less soluble form (Economic Microbiology, Vo. 2, p. 315, Academic Press).

An example of this method is described in detail in Japan Patent Publication No. 10155/1978, according to which riboflavin is converted into reduced-form riboflavin having low solubility by the addition of a hydrosulfite and precipitated as crude crystals. The obtained crystals are oxidized in an acidic suspension and purified by recrystallization. Purified riboflavin can be prepared by his method, but the yields were too low for practical use. According to Example 1 of Japan Patent Publication No. 13276/1982, the heated culture solution from which bacteria have been removed is concentrated and reduced with titanium trichloride to form a precipitate. The precipitate is oxidized in air and purified by dissolution in hydrochloric acid and alkali precipitation (29 % yield after purification). It is also known to extract the product using butanol or other organic solvents in the treatment of reduced-form (leuco) riboflavin (U. S. Pat. No. 2,464,243).

It is the object of the present invention to provide an improved process for the cultivation of riboflavin producing yeast preserved an agar medium, said yeast being preliminarily cultivated in a liquid medium, by which the productivity of riboflavin is remarkably improved and the inhibition by iron ions is prevented.

The invention to accomplish this object comprises a process for the preparation of riboflavin, characterized by planting a yeast belonging to the genus Saccbaromyces and having riboflavin-producing ability, after pre-cultivation in liquid, on a riboflavin-producing culture medium containing zinc ions.

### Microorganisms to be Used

In order to prepare riboflavin by cultivation, it is necessary to use a microorganism suitable for the purpose. In the following, microorganisms which are suitable for the process according to the present invention, are described.
The strain disclosed in the literature by some of the present inventors relating to a process for the preparation of riboflavin by fermentation using acetic acid as carbon source (see Agr. Biol. Chem. Vol. 28, pp. 559, 566 & 765 (1964)) is one of the examples. The microorganism was designated in said literature as Candida robusta, but as spores were found in the type strain of Candida robusta later, Candida robusta is re-classified as Saccharomyces cerevisiae in J. Lodder's The Yeast (1970).

However, as no formation of spores was recognized in the strain discussed in said literature, it is assumed that the strain is a non-spore type of Saccharomyces cerevisiae. The strain is hereinafter referred to as Saccharomyces cereviciae (Candida robusta) in the present specification.

Other appropriate strains include, for example, mutants derived from Saccharamyces cerevisiae (Candida robusta AHU 3405).
Further, according to the present invention a riboflavin-producing fungus belonging to the genus Saccharomyces and having purine requirements may be used. Any microorganism that has these characteristics can be used. What distinguishes the present microorganism from those employed in the prior art is purine requirement. An example of the appropriate strains is Saccharomyces cerevisiae P-154 (FERM BP-566), which is a purine-requiring mutant derived from Saccharomyces cerevisiae (Candida robusta AHU 3405). The strain of Saccharomyces cerevisiae P-154 can be relatively easily obtained by subjecting a parent strain of a riboflavin-producing yeast belonging to the genus Saccharomyces to ordinary mutation treatment.

More particularly, Saccharomyces cerevisiae (Candida robusta AHU 3405) (listed as one of the preserved microorganisms of the Faculty of Agriculture, Hokkaido Univ.), selected as parent strain, may be irradiated with ultraviolet ray or treated with an agent such as N-methyl-N'-nitro-N-nitrosoguanidine and then smeared on a yeast extract-malt agar medium. The desired mutant is selected from the grown colonies by the following method: the replica of said colonies is formed on a culture medium containing the minimum medium having the composition shown in Table 1 and 0.005 % of a purine compound such as adenine, and such colony is selected as purin-requiring mutant that cannot be grown in the minimum medium but can be grown in the medium containing a purine compound.

**Table 1**

| Composition of Minimum Medium | |
|---|---|
| Ingredient | Concentration |
| glucose | 20 g/ℓ |
| (NH₄)₂SO₄ | 2 g/ℓ |
| MgSO₄·7H₂O | 0.5 g/ℓ |
| KH₂PO₄ | 1.5 g/ℓ |
| biotin | 2 µg /ℓ |
| agar | 15 g/ℓ |
| pH | 6.0 |

The growth test of the obtained mutant on purine compounds is conducted to examine the purine requirements of the mutant according to the following method. Saccharomyces cerevisiae (Candida robusta AHU 3405), the parent strain, and the mutant derived therefrom are cultivated for 24 hours in a nutritious liquid medium having the composition shown in Table 2, and washed with physiological saline. The suspensions of the cultures are each inoculated on 5 mℓ portions of the minimum medium to which various amounts of purines shown in Table 3 had been added. After the cultivation is continued for 5 days at 30°C, the growth rate of the strains is measured based on the absorbance at 610 nm. The relative growth rate of the mutant, with the growth rate of the parent strain being 100, is shown in Table 3. The minimum medium to which purine compounds in amounts listed in Table 3 is added has the same composition as shown in Table 1 except that agar and biotin were omitted and that 103 g/ℓ of calcium acetate were added in place of glucose.

It is confirmed from Table 3 that Saccharomyces cerevisiae P-154 was endowed with purine requirements.

**Table 2**

| Nutritious Liquid Medium | |
|---|---|
| Ingredient | Concentration |
| glucose | 20 g/ℓ |
| yeast extract | 3 g/ℓ |
| malt extract | 3 g/ℓ |
| polypeptone | 5 g/ℓ |
| pH | 6.0 |

**Table 3**

| Relative Growth Rate | | |
|---|---|---|
| Strain | Purine concn.(%) | Relative growth rate |
| Saccharomyces cereviciae (Candida robusta AHU 3405) | 0 | 100 |
| | adenine 0.1 | 100 |
| Saccharomyces cereviciae P-154 | 0 | 0 |
| | adenine 0.01 | 129 |
| | adenine 0.1 | 94 |
| | hypoxanthine 0.1 | 94 |

### (3-Amino-1,2,4-triazole-resistant Mutants)

Other suitable miccoorganisms include any riboflavin-producing yeast belonging to the genus Saccharomyces and having resistance to 3-amino-1,2,4-triazole. They are distinguished from those employed in the prior art in the resistance to 3-amino-1,2,4-triazole. Saccharomyces cerevisiae TW-573 (FERM BP-567), and Saccharomyces cerevisiae TP-1010 (FERM BP-565), which are 3-amino-1,2,4-triazole-resistant mutants derived from Saccharomyces cerevisiae (Candida robusta AHU 3405) and Saccharomyces cerevisiae P-154 (FERM BP-566), respectively, are the examples of the suitable strains.

These strains can be obtained relatively easily by subjecting parent strains of riboflavin-producing yeast belonging to the genus Saccharomyces to ordinary mutation treatment.

More particularly, the same parent strain as that shown in the example of the preparation of the purine-requiring mutant is subjected to the same mutation treatment and then smeared on the agar medium having the composition shown in Table 1 and further containing CaCℓ₂·2H₂O (0.3 g/ℓ) and 3-amino-1,2,4-triazole in an amount which prevents the growth of the parent strain. The grown colony is selected as 3-amino-1,2,4-triazole-resistant mutant.

When the parent strain requires nutrients, the ingredients required are further added to the medium having the above-described composition, and thereafter the 3-amino-1,2,4-triazole-resistant mutant can be selected in the same manner as above.

The growth test of the obtained mutants on 3-amino-1,2,4-triazole is conducted to examine the resistance to 3-amino-1,2,4-triazole of the mutants, according to the following method: the parent strain and the 3-amino-1,2,4-triazole-resistant mutants are washed with physiological saline and the suspensions of the strains are each inoculated in 5 mℓ portions of the medium listed in Table 1, to which 3-amino-1,2,4-triazole in amounts listed in Table 4 has been added. After the cultivation is continued for 2 days at 30°C, the growth rate of the strains is measured based on the absorbance at 610 nm. The relative growth rate of the strains, with the growth rate of the strains to which no 3-amino-1,2,4-triazole is added being 100, is shown in Table 4. The culture medium having the composition of Table 1 to which 0.005 % of adenine are added is used for Saccharomyces cerevisiae P-154 and Saccharomyces cerevisiae TP-1010 derived therefrom.

**Table 4**

| Relative Growth Rate | | |
|---|---|---|
| Strain | 3-Amino-1,2,4-triazole concn. (mM) | Relative growth rate |
| Saccharomyces cerevisiae (Candida robusta AHU3405) | 0 | 100 |
| | 1 | 1 |
| | 5 | 0 |
| | 10 | 0 |
| Saccharomyces cerevisiae TW-573 | 0 | 100 |
| | 1 | 60 |
| | 5 | 19 |
| | 10 | 1 |
| Saccharomyces cerevisiae P-154 | 0 | 100 |
| | 20 | 22 |
| | 40 | 20 |
| | 80 | 20 |
| | 100 | 9 |
| Saccharomyces cerevisiae TP-1010 | 0 | 100 |
| | 20 | 111 |
| | 40 | 92 |
| | 80 | 49 |
| | 100 | 17 |

It is confirmed from Table 4 that Saccharomyces cerevisiae TW-573 and Saccharomyces cerevisiae TP-1010 are endowed with the resistance to 3-amino-1,2,4-triazole.

If it is attempted to separate riboflavin by crystalllzation, a preferred microorganism is the one that requires relatively small nutrients, produces riboflavin without addition of meat extract, polypeptone, corn steep liquor or other substances having a complicated composition, and does not produce large amounts of by-products other than riboflavin, to avoid large amounts of substances affecting the crystallization of riboflavin. The microorganisms described above satisfy these conditions, too.

### (Separation)

The culture in which riboflavin is formed with the substrate, medium ingredients, and the microorganism selected according to the standards described above has a less complicated composition than those of the prior art, thus making it possible to prepare more purified riboflavin crystals in higher yields, followed by the separation of the solid matters such as yeast cells or calcium carbonate from the heated aqueous solution, and the crystallization of the product from the aqueous solution by cooling or other ordinary method.

The culture from which the crystals are to be separated may be in a state of liquid obtained after fermentation (broth) or of solid matter obtained by cooling and filtering or centrifuging the solution after fermentation, in which yeast cells and riboflavin crystals are mixed. As at least part of the formed riboflavin is considered to be embedded in the cells, it must be extracted with hot water, and separated together with those existing outside the cells in the form of heated aqueous solution from yeast cell and other solid matters. The conditions for the extraction with water are determined considering the stability and solubility of riboflavin. Usually, the extraction is carried out under acidic conditions at a temperature not lower than 50°C, preferably not lower than 60°C. Water overheated, for example, at 120°C can be used under pressures. The amount of the water must be sufficient for dissolving the riboflavin contained in the culture and varies according to the temperature of the heated aqueous solution. Therefore, water is usually added not only when solid matters are used but also when the culture solution itself is used.

Although riboflavin crystals are obtained when the heated aqueous solution is simply cooled, the liquid extract may be optionally concentrated (while separating calcium carbonate or the like, if formed) and then crystallized.

Riboflavin crystals having higher purity can be obtained by optional recyrstallization using water, aqueous acetic or hydrochloric acid solution, or other solvents.

### Cultivation in a Zinc-containing Medium

In the report mentioned in the foregoing paragraph (Takao, 1964), riboflavin was prepared by directly inoculating a yeast preserved on an agar medium to a riboflavin-producing medium. The effect of metal ions was then examined by the addition of 0.1 mg/ℓ, 1 mg/ℓ, and 5 mg/ℓ portions of various metal ions. As the result, Bi, Li, and Mn ions were found to be a little effective, while Fe, Ag, Cu, and Hg ions were found to prevent the preparation of riboflavin.

It was reported that zinc-containing samples showed the same values as those to which the metal had not been added (12.1 to 12.8 mg/100 mℓ). The present inventors, however, unexpectedly found that the addition of a trace amount of zinc to a medium for the production of riboflavin, in which a yeast grown on an agar medium was to be inoculated, not directly but after pre-cultivation in a liquid medium, remarkably improved the productivity of riboflavin, and also decreased the adverse effect due to iron ions, which leads to the accomplishment of this invention.

In more detail, the invention comprises inoculating a riboflavin-producing yeast grown on an agar medium, for example, Saccharomyces cerevisiae (Candida robusta AHU 3402) or Saccharomyces cerevisiae (Candida robusta AHU 3405) on a liquid medium for pre-cultivation, which contains glucose, yeast extract, polypeptone, and the like, cultivating the yeast under shaking for 1 to 2 days at 30°C, and when the yeast has been grown sufficiently, inoculating a predetermined amount of the preculture on a riboflavin-producing medium directly or after washing with physiological saline.

It is preferable to inoculate the pre-culture solution to the riboflavin-producing medium in an amount of 3 % or more. The greater amount of the inoculated pre-culture brings about the higher riboflavin productivity. For example, when 3.8 % of the pre-culture was inoculated to a medium containing 0.5 mg/ℓ of zinc, 0.92 g/ℓ of riboflavin was obtained after 6 day cultivation, while when 2.5 % of the pre-culture was inoculated, riboflavin was obtained in an unsatisfactory yield of 0.38 g/ℓ.

However, inoculating in too large amounts is also disadvantageous. The amount of the inoculated pre-culture is preferred to be 25 % or less.

A process for the cultivation of a riboflavin-producing microorganism on a lower aliphatic compound as carbon source will be described in the examples.

Nitrogen compounds in various forms can be used as nitrogen source. Inorganic nitrogen compounds are preferred and they include, for example, ammonium sulfate, ammonium chloride, and ammonium carbonate. The use of large amounts of polypeptone, or other organic nitrogen sources may adversely affect the crystallization.

Potassium dihydrogen phosphate, magnesium sulfate, or other inorganic salts are further incorporated besides said carbon and nitrogen sources. When purine-requiring strains are used, purine compounds such as adenine, mineral acid salts of adenine, adenosine, adenylic acid, ribonucleic acid, and further hypoxanthine, inosine and the like are added. Moreover, optional incorporation of vitamines such as biotin, micronutrients such as amino acids or nucleic acid bases may increase the amount of riboflavin accumulated.

The cultivation method described above is appliable to the cultivation of mutants. Carbon and nitrogen sources can be selected from among a broader range, including glucose, sucrose, xylose, and other saccharides, or amino acids, polypeptone, and other organic nitrogen compounds. When these compounds are used, however, riboflavin must be prepared by a method other than crystallization as described above.

Zinc ions can be added in the form of zinc sulfate, chloride, or acetate. The concentration of the zinc ions to be added may be determined on scrutinizing the effect, but the preferable amount of the zinc ions may be within the range of 0.1 to 100 mg/ℓ. Small amounts of 0.05 mg/ℓ or less are not effective, while too large amounts, for example, exceeding 300 mg/ℓ of zinc ions are not preferable because the growth of the yeast and the utilization rate of the carbon source are considerably decreased.

The most desirable concentration of the zinc ions is varied in dependence on the concentration of the iron ions contained in the medium. For example, when the amount of iron ions is 0.1 mg/ℓ or less, 0.5 mg/ℓ of zinc ions will suffice. However, when 5 mg/ℓ of iron ions are contained, it is necessary to add 10 to 30 mg/ℓ of zinc ions.

Aerobic conditions are preferred for the cultivation. The pH value of the culture medium is from 2 to 10, preferably from 6 to 9 for most desirable results. The temperature is from the range of 20 to 37°C considering the appropriateness for the growth of the strains used and for the production of riboflavin.

Shaking culture, submerged aeration-agitation culture, or other methods are employed.

The present invention will be more readily understood by the following examples. The microorganisms used in the examples were pre-cultivated under the following conditions and then inoculated in the fermentation medium in an amount of 12.6 %.

### Pre-cultivation conditions:

inoculated in 100 mℓ of a pre-culture medium containing:
2 % glucose,
0.5 % polypeptone,
0.3 % yeast extract, and
0.3 % malt extract
shaking cultivation at 30°C for 30 hours.

### Fundamental composition of fermentation medium:

| | |
|---|---|
| calcium acetate | 103 g/ℓ |
| (NH₄)₂SO | 3 g/ℓ |
| KH₂PO₄ | 2 g/ℓ |
| MgSO₄·7H₂O | 1 g/ℓ |
| ZnSO₄·7H₂O | 2.2 mg/ℓ |
| pH | 7.0 |

### Example 1

3 ℓ of a fermentation medium having the fundamental composition as shown above, with the addition of 1 g/ℓ of adenine, was charged in a 7-ℓ jar fermenter, and heated at 120°C for 20 minutes. Saccharomyces cerevisiae P-154 was inoculated in the medium and subjected to aeration-agitation culture at 30°C for 6 days (0.5 v.v.m., 400 rpm). 1.45 g/ℓ of riboflavin was accumulated in the culture liquid obtained after cultivation.

700 mℓ portion of this culture liquid was cooled and centrifuged to precipitate a mixture of yeast cells and riboflavin crystals. 1 ℓ of water was added to the precipitate, and extracted at 80°C for 1.5 hour. After the heated liquid extract was cooled, 513 mg of riboflavin crystals having a purity of 95.3 % were obtained. The crystals were recrystallized from dilute acetic acid, yielding riboflavin crystals of purity of 98 % or more.

### Example 2

300 g of water was added to another 670 mℓ portion of the culture solution of Example 1, to which was further added hydrochloric acid to adjust the pH to 6.0. The solution was maintained at 80°C for 1.5 hour and filtered under heating to obtain 850 g of a liquid extract. The liquid extract was concentrated 5 times and cooled to form 2.18 g of crude crystals containing riboflavin and calcium carbonate.

90 g of a 0.44 % aqueous acetic acid solution was added to a 480 mg portion of these crude crystals. The solution was heated at 95°C for 4 hours and, after filtering off the insoluble matters, the filtrate was cooled to yield 146 mg of riboflavin crystals having a purity of 99 % or more.

### Example 3

Saccharomyces cerevisiae TW-573 was cultivated in the same manner as described in Example 1 except that the fermentation medium had the fundamental composition.

1.2 ℓ of the culture (the amount of riboflavin being 1.47 g/ℓ) was cooled and centrifuged to precipitate a mixture of yeast cells and riboflavin crystals. 2 ℓ of water was added to the precipitate and extracted at 80°C for 1.5 hours. The heated liquid extract was concentrated into a volume of 1 ℓ and then cooled, yielding 895 mg of riboflavin crystals having a purity of 97.3 %.

A 740 mℓ portion of the filtrate was further concentrated 3.3 times, yielding 388 mg of riboflavin crystals having a purity of 96.0 %.

### Example 4

Saccharomyces cerevisiae P-154 was cultivated under shaking at 30°C for 6 days in the same medium as used in Example 1. The amount of the riboflavin accumulated in the culture liquid was 1.55 g/ℓ.

When Saccharomyces cerevisiae (Candida robusta AHU 3405), the parent strain of Saccharomyces cerevisiae P-154, was used for comparison, the amount of the riboflavin accumulated in the culture liquid was 0.85 g/ℓ.

### Example 5

Saccharomyces cerevisiae P-154 was cultivated in the same manner as in Example 4, except that hypoxanthine was used in place of adenine in the same amount. The strain accumulated 1.48 g/ℓ of riboflavin in the culture liquid.

### Example 6

Saccharomyces cerevisiae P-154 was subjected to liquid pre-cultivation in the same manner as in Example 4, and then cultivated under shaking at 30°C for 6 days to prepare riboflavin, under the same conditions as in Example 4 except that ammonium sulfate was incorporated in the fermentation medium in an amount of 3.8 g/ℓ and the concentration of zinc was varied as follows. The results are shown in Table 5.

**Table 5**

| Zinc concn. (mg/ℓ) | Riboflavin (g/ℓ) |
|---|---|
| 0 | 0.85 |
| 0.5 | 1.40 |
| 10 | 1.41 |
| 30 | 1.35 |

### Example 7

Saccharomyces cerevisiae TW-573 was cultivated under shaking at 30°C for 9 days to prepare riboflavin in the fermentation medium having the same composition as that of Example 3 except that the amount of ammonium sulfate was 3.8 g/ℓ and the zinc concentration was varied. The results are shown in Table 6.

**Table 6**

| Zinc concn. (mg/ℓ) | Riboflavin (g/ℓ) |
|---|---|
| 0 | 0.75 |
| 0.1 | 1.02 |
| 0.5 | 1.21 |
| 2.5 | 1.29 |
| 5 | 1.32 |
| 10 | 1.39 |
| 20 | 1.36 |
| 30 | 1.36 |
| 100 | 1.32 |

### Example 8

Saccharomyces cerevisiae TP-1010 was cultivated in the same manner as in Example 3, except that 0.1 % of adenine was added to the fermentation medium of the fundamental composition. TP-1010 accumulated 2.50 g/ℓ of riboflavin in the culture liquid.

### Example 9

Saccharomyces cerevisiae TP-1010 was subjected to liquid pre-cultivation in the same manner as in Example 3 and cultivated at 30°C for 10 days to prepare riboflavin. The composition of the fermentation medium was the same as that of Example 8 except that the amount of ammonium sulfate was 3.8 g/ℓ and the zinc concentration was varied. The results are shown in Table 7.

**Table 7**

| Zinc concn. (mg/ℓ) | Riboflavin (g/ℓ) |
|---|---|
| 0 | 1.62 |
| 0.5 | 2.17 |
| 2.5 | 2.18 |
| 5 | 2.26 |
| 10 | 2.20 |
| 30 | 2.22 |

### Example 10

Saccharomyces cerevisiae (Candida robusta AHU 3405) was inoculated on a potato dextrose agar medium (manufactured by Nissui Seiyaku) and cultured at 30°C for 24 hours.

The grown yeast was inoculated in a liquid medium containing 2 % of glucose, 0.5 % of polypeptone, 0.3 % of yeast extract, and 0.3 % of malt extract, and subjected to rotation-shaking culture at 190 rpm at 30°C for 25 hours. The pH value of the culture liquid was 4.3, and the absorbance at 610 nm was 5.7. The riboflavin-producing medium composition was 10.3 % of calcium acetate as carbon source, 0.38 % of ammonium sulfate, 0.2 % of potassium dihydrogen phosphate, and 0.1% of magnesium sulfate and zinc sulfate was further added to the medium in an amount of 0.1 to 300 mg/ℓ in terms of zinc ion concentration.

4.72 mℓ of this medium, the pH value being adjusted to 7.0, was charged in a test tube of 21 mm in diameter, whereto 0.28 mℓ of said pre-culture solution was inoculated and subjected to reciprocal shaking culture at 220 rpm at 30°C for 8 days. The amount of the riboflavin in the culture liquid was calculated based on the absorbance of the centrifuged filtrate at 450 nm. The amount of the strain was determined based on the absorbance at 610 nm, and acetic acid was analyzed by means of high-performance liquid chromatography using an ion-exchange resin. As understood from the results shown in Table 8, the riboflavin productivity was remarkably increased by the addition of 0.1 to 100 mg/ℓ of zinc. Similar results were obtained from another series of experiments in which the length of the cultivation days was varied. Those with marks a and b in Table 8 refer to part of those data, which were obtained after cultivation for 6.7 days (160 hours) and 4 days each.

**Table 8**

| Effects of Zinc on the Production of Riboflavin | | | |
|---|---|---|---|
| Zn⁺⁺ concn. (mg/ℓ) | riboflavin (g/ℓ) | cell growth (OD_{610 nm}) | residual acetic acid (g/ℓ) |
| 0 | 0.46 | 14.8 | 5.7 |
| 0.1 | 0.92 | 11.6 | 3.4 |
| 0.2 | 1.00 | 10.3 | 2.2 |
| 0.3 | 0.98 | 9.5 | 7.3 |
| 0.4 | 1.01 | 8.6 | 5.7 |
| 0.5 | 0.99 | 9.0 | 9.9 |
| 0.75a | 0.75 | 11.3 | 6.7 |
| 1.0 a | 0.83 | 11.4 | 6.5 |
| 3.0 a | 0.79 | 11.3 | 6.6 |
| 6.0 b | 0.78 | 16.9 | 0 |
| 10 b | 0.78 | 17.3 | 0 |
| 30 | 0.80 | 8.0 | 25.2 |
| 100 | 0.77 | 7.5 | 29.4 |
| 300 | 0.31 | 3.9 | 65.3 |

### Example 11

Glucose, sucrose, glycerol, ethanol, and calcium gluconate were added in place of calcium acetate in Example 10. Calcium carbonate was added in an amount of 70 % of the concentration of the carbon source in the case of the carbon sources except calcium gluconate to prevent the decrease in pH values. Biotin was also added to these carbon sources in an amount of 1 µg/ℓ. As understood from Table 9, the effect of zinc was recognized with these carbon sources as well.

**Table 9**

| Effects of Zinc with Various Carbon Sources | | | | |
|---|---|---|---|---|
| Carbon source | Carbon source concn.(%) | Zn⁺⁺ concn. (mg/ℓ) | riboflavin (mg/ℓ) | cell growth (OD_{610 nm}) |
| glucose | 7 | 0 | 58 | 19.1 |
| | 7 | 0.5 | 116 | 10.0 |
| | 1.75 | 0 | 11 | 9.5 |
| | 1.75 | 0.5 | 51 | 6.3 |
| sucrose | 1.75 | 0 | 23 | 11.9 |
| | 1.75 | 0.5 | 66 | 4.5 |
| glycerol | 1.75 | 0 | 19 | 15.7 |
| | 1.75 | 0.5 | 36 | 10.8 |
| ethanol | 1.75 | 0 | 17 | 8.4 |
| | 1.75 | 0.5 | 24 | 5.8 |
| calcium gluconate | 7 | 0 | 51 | 20.8 |
| | 7 | 0.5 | 109 | 23.5 |

### Example 12

The examination was made on the effects of the addition of zinc ions in the presence of iron ions by cultivating the strains under the same conditions as in Example 10 except that ferrous sulfate was added to the riboflavin-producing medium in an amount of 5 mg/ℓ in terms of iron ion concentration. As understood from Table 10, addition of zinc ion in an amount of 10 to 30 mg/ℓ leads to satisfactory riboflavin production even in the presence of 5 mg/ℓ of iron ions.

**Table 10**

| Effects of Zinc in the Presence of Iron Ions | | | |
|---|---|---|---|
| Fe⁺⁺ (mg/ℓ) | Zn⁺⁺ (mg/ℓ) | riboflavin (g/ℓ) | cell growth (OD₆₁₀ₙₘ) |
| 5 | 0 | 0.12 | 20.6 |
| 5 | 0.5 | 0.14 | 16.2 |
| 5 | 10 | 0.81 | 15.0 |
| 5 | 30 | 0.94 | 14.8 |
| 5 | 100 | 0.09 | 15.0 |
| 5 | 300 | 0.09 | 14.8 |
| 0.1 or less | 0.5 | 0.95 | 16.5 |

### Example 13

Saccharomyces cereviciae (Candida robusta AHU 3402) was subjected to the same experiment as described in Example 10. The amounts of riboflavin produced and cell growth after 6 day cultivation are shown in Table 13. The effects of the addition of zinc was also recognized in the present strain.

**Table 13**

| Zn⁺⁺ (mg/ℓ) | riboflavin (g/ℓ) | cell growth (OD_{610 nm}) |
|---|---|---|
| 0 | 0.32 | 19.9 |
| 0.5 | 0.51 | 19.3 |

## Claims

1. A process for the preparation of riboflavin, which comprises preliminarily cultivating a yeast belonging to the genus Saccharomyces and having riboflavin-producing ability in liquid and then cultivating said pre-cultured product in a riboflavin-producing medium containing zinc ions.

2. A process for the preparation of riboflavin as set forth in claim (1), wherein the zinc concentration of said riboflavin-rpoducing medium is not lower than 0.1 mg/ℓ and not higher than 100 mg/ℓ.

## Patentansprüche

1. Verfahren zum Herstellen von Riboflavin, das das vorbereitende Kultivieren einer zur Gattung Saccharomyces gehörenden Hefe mit der Fähigkeit zur Riboflavinerzeugung in einer Flüssigkeit und das Kultivieren des vorkultivierten Produktes in einem Riboflavin erzeugenden Medium, das Zinkionen enthält, umfaßt.

2. Verfahren zum Herstellen von Riboflavin nach Anspruch 1, wobei die Zinkkonzentration des Riboflavin erzeugenden Mediums nicht niedriger als 0,1 mg/l und nicht höher als 100 mg/l ist.

## Revendications

1. Procédé de préparation de riboflavine, selon lequel on cultive préalablement une levure appartenant au genre Saccharomyces et ayant la capacité de produire de la riboflavine dans un liquide, et on cultive ensuite ce produit pré-cultivé dans un milieu producteur de riboflavine contenant des ions zinc.

2. Procédé de préparation de riboflavine selon la revendication 1, dans lequel la concentration en zinc du milieu producteur de riboflavine n'est pas inférieure à 0,1 mg/l et non supérieure à 100 mg/l.
